(19) **Europäisches Patentamt**
**European Patent Office**
**Office européen des brevets**

(11) **EP 2 341 823 B1**

(12) **EUROPEAN PATENT SPECIFICATION**

(45) Date of publication and mention
of the grant of the patent:
**15.06.2016 Bulletin 2016/24**

(51) Int Cl.:
*A61B 5/00* (2006.01)      *G02B 27/26* (2006.01)
*G01J 9/02* (2006.01)      *G05F 1/00* (2006.01)
*G01N 21/21* (2006.01)      *G01B 9/02* (2006.01)

(21) Application number: **09825421.2**

(22) Date of filing: **05.11.2009**

(86) International application number:
**PCT/US2009/063420**

(87) International publication number:
**WO 2010/054097 (14.05.2010 Gazette 2010/19)**

(54) **SYSTEM AND METHOD FOR PROVIDING FULL JONES MATRIX-BASED ANALYSIS TO DETERMINE NON-DEPOLARIZING POLARIZATION PARAMETERS USING OPTICAL FREQUENCY DOMAIN IMAGING**

SYSTEM UND VERFAHREN ZUR BEREITSTELLUNG VON VOLLSTÄNDIG AUF JONES-MATRIZEN BERUHENDEN ANALYSEN ZUR BESTIMMUNG NICHT ENTPOLARISIERENDER POLARISATIONSPARAMETER MITHILFE DER ABBILDUNG VON OPTIKFREQUENZDOMÄNEN

SYSTÈME ET PROCÉDÉ POUR RÉALISER UNE ANALYSE À BASE DE MATRICE DE JONES COMPLÈTE AFIN DE DÉTERMINER DES PARAMÈTRES DE POLARISATION NON DÉPOLARISANTS À L'AIDE D'UNE IMAGERIE DANS LE DOMAINE FRÉQUENTIEL OPTIQUE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **05.11.2008 US 111479 P**

(43) Date of publication of application:
**13.07.2011 Bulletin 2011/28**

(73) Proprietor: **The General Hospital Corporation Boston, MA 02114 (US)**

(72) Inventors:
• **DE BOER, Johannes, F.**
**NL-1186 DC Anstelveen (NL)**
• **PARK, Boris, Hyle**
**Somerville**
**MA 02143 (US)**
• **KIM, Ki Hean**
**Gyeongbuk 790-751 (KR)**

(74) Representative: **Quinterno, Giuseppe**
**Jacobacci & Partners S.p.A.**
**Corso Emilia 8**
**10152 Torino (IT)**

(56) References cited:
US-A1- 2007 024 860      US-A1- 2007 038 040
US-A1- 2007 236 700      US-A1- 2008 007 734

• W.Y. OH ET AL: "High-speed polarization sensitive optical frequency domain imaging with frequency multiplexing", OPTICS EXPRESS, vol. 16, no. 2, 1 January 2008 (2008-01-01), page 1096, XP055056859, ISSN: 1094-4087, DOI: 10.1364/OE.16.001096
• PARK B H ET AL: "Jones matrix analysis for a polarization-sensitive optical coherence tomography system using fiber-optic components", OPTICS LETTERS, OSA, OPTICAL SOCIETY OF AMERICA, WASHINGTON, DC, US, vol. 29, no. 21, 1 November 2004 (2004-11-01), pages 2512-2514, XP002375104, ISSN: 0146-9592, DOI: 10.1364/OL.29.002512
• MASAHIRO YAMANARI ET AL: "Polarization-sensitive swept-source optical coherence tomography with continuous source polarization modulation", OPTICS EXPRESS, vol. 16, no. 8, 14 April 2008 (2008-04-14), page 5892, XP055056861, ISSN: 1094-4087, DOI: 10.1364/OE.16.005892

**Description**

FIELD OF THE DISCLOSURE

The present disclosure relates to optical polarization-sensitive optical coherence tomography apparatus and methodsfor optical frequency domain imaging (e.g., partially fiber-based) to obtain information associated with an anatomical structure or a sample, and more particular wherein the evolution of the polarization state of the sample arm light is used to determine the non-depolarizing polarization parameters of the sample.

BACKGROUND INFORMATION

US 2007/024860A1 discloses an apparatus comprising one first arrangement configured to provide at least one first electro-magnetic radiation, wherein a frequency of radiation provided by the at least one first arrangement is variable over time, and at least one second arrangement configured to split the at least one first electro-magnetic radiation into second and third radiations having different orthogonal states and to apply one first characteristic to the second radiation and a second characteristic to the third radiation, the first and second characteristics being different from one another.

US 2007/024860A1 also discloses a method comprising providing at least one first electromagnetic radiation, wherein a frequency of radiation associated with the at least one first radiation varies over time, and splitting the at least one first radiation into second and third radiations having different orthogonal states and applying one first characteristic to the second radiation and a second characteristic to the third radiation, the first and second characteristics being different from one another.

"High-speed polarization sensitive optical frequency domain imaging with frequency multiplexing" by W.Y.Oh et al in Optics Express, Vol.16, No.2 of 21.01.2008 discloses a polarization-sensitive FD-OCT apparatus with simultaneous detection of different polarization components.

Optical coherence tomography ("OCT") is an imaging technique that can measure an interference between a reference beam of light and a beam reflected back from a sample. A detailed system description of traditional time-domain OCT is described in Huang et al., "Optical Coherence Tomography," Science 254, 1178 (1991). Optical frequency domain imaging ("OFDI") techniques, which can be also known as swept source or Fourier-domain optical coherence tomography (OCT) techniques, can be OCT procedures which generally use swept laser sources. For example, an optical beam is focused into a tissue, and the echo time delay and amplitude of light reflected from tissue microstructure at different depths are determined by detecting spectrally resolved interference between the tissue sample and a reference as the source laser wavelength is rapidly and repeatedly swept. A Fourier transform of the signal generally forms an image data along the axial line (e.g., an A-line). A-lines are continuously acquired as the imaging beam is laterally scanned across the tissue in one or two directions that are orthogonal to the axial line.

The resulting two or three-dimensional data sets can be rendered and viewed in arbitrary orientations for gross screening, and individual high-resolution cross-sections can be displayed at specific locations of interest This exemplary procedure allows clinicians to view microscopic internal structures of tissue in a living patient, facilitating or enabling a wide range of clinical applications from disease research and diagnosis to intraoperative tissue characterization and image-guided therapy Exemplary detailed system descriptions for spectral-domain OCT and Optical Frequency Domain Interferometry are described in WO03/062802A2 and U S Patent Application Serial No 60/514,769, respectively.

A contrast mechanism in the OFDI techniques can generally be an optical back reflection originating from spatial reflective-index variation in a sample or tissue The result can be a so-called an "intensity image" that may indicate the anatomical structure of tissue up to a few millimeters in depth with spatial resolution ranging typically from about 2 to 20 $\mu$m While the intensity image can provide a significant amount of morphological information, birefringence in tissues may offer another contrast useful in several applications such as quantifying the collagen content in tissue and evaluating disease involving the birefringence change in tissue. Polarization-sensitive OCT can provide an additional contrast by observing changes in the polarization state of reflected light. The first fiber-based implementation of polarization-sensitive time-domain OCT is described in Saxer et al., "High-speed fiber-based polarization-sensitive optical coherence tomography of in vivo human skin," Opt. Lett. 25, 1355 (2000).

In polarization-sensitive time-domain OCT techniques, a simultaneous detection of interference fringes in two orthogonal polarization channels can facilitate a complete characterization of a reflected polarization state, as described in J.F. de Boer et al., "Determination of the depth-resolved Stokes parameters of light backscattered from turbid media by use of polarization-sensitive optical coherence tomography," Opt. Lett. 24, 300

(1999). There can be two non-depolarizing polarization parameters: birefringence, characterized by a degree of phase retardation and an optic axis orientation, and diattenuation, which can be related to dichroism and characterized by an amount and an optic axis orientation. Together, these optical properties may be described by, e.g., the 7 independent parameters in the complex 2x2 Jones matrix.

The polarization state reflected from the sample can be compared to the state incident on the sample quite easily in a bulk optic system, as the polarization state incident on the sample can be controlled and fixed. However, an optical fiber may have a significant disadvantage in that a propagation through optical fiber can alter the polarization state of light. In this case, the polarization state of light incident on the sample may not be easily controlled or determined. In addition, the polarization state reflected from the sample may not be necessarily the same as the polarization state received at the detectors. Assuming negligible diattenuation, or polarization-dependent loss, optical fiber changes the polarization states of light passing through such fiber in such a manner as to preserve the relative orientation between states. The overall effect of propagation through optical fiber and non-diattenuating fiber components can be similar to an overall coordinate transformation or some arbitrary rotation. In other words, the relative orientation of polarization states at all points throughout propagation can be preserved, as described in U.S. Patent No. 6,208,415.

There have been a number of approaches that can take advantage to determine the polarization properties of a biological sample imaged with polarization-sensitive OCT. Such approaches have suffered from some disadvantage, however.

For example, a vector-based method has been used to characterize birefringence and optic axis orientation only by analyzing rotations of polarization states reflected from the surface and from some depth for two incident polarization states perpendicular in a Poincaré sphere representation as described in the Saxer Publication, J.F. de Boer et al., "Determination of the depth-resolved Stokes parameters of light backscattered from turbid media by use of polarization-sensitive optical coherence tomography," Opt. Lett. 24, 300

(1999), and B.H. Park et al., "In vivo burn depth determination by high-speed fiber-based polarization sensitive optical coherence tomography," J. Biomed. Opt. 6,474 (2001).

Mueller matrix based methods are capable of determining birefringence, diattenuation, and optic axis orientation as described in S.L. Jiao et al., "Two-dimensional depth-resolved Mueller matrix of biological tissue measured with double-beam polarization-sensitive optical coherence tomography," Opt. Lett. 27, 101 (2002), S. Jiao et al., "Optical-fiber-based Mueller optical coherence tomography," Opt. Lett. 28, 1206 (2003), and S.L. Jiao et al., "Depth-resolved two-dimensional Stokes vectors of backscattered light and Mueller matrices of biological tissue measured with optical coherence tomography," Appl. Opt. 39, 6318 (2000). These typically utilize a multitude of measurements using a combination of incident states and detector settings and limits their practical use for in vivo imaging.

Jones matrix based approaches have also been used to characterize the non-depolarizing polarization properties of a sample as described in S. Jiao et al., "Optical-fiber-based Mueller optical coherence tomography," Opt. Lett. 28, 1206 (2003) and S.L. Jiao and L.V. Wang, "Jones-matrix imaging of biological tissues with quadruple-channel optical coherence tomography," J. Biomed. Opt. 7, 350 (2002). The description of these approaches has limited a use of optical fiber and fiber components such as circulators and fiber splitters such that these components must be traversed in a round-trip fashion and assumes that sample birefringence and diattenuation share a common optic axis. These approaches can use a multitude of measurements using a combination of incident states and detector settings and limits their practical use for in vivo imaging.

Generally, in nearly all of polarization sensitive time domain, Spectral Domain OCT, or OFDI systems, the polarization properties can be measured using different incident polarization states on the sample in a serial manner, i.e. the incident polarization state incident on the sample was modulated as a function of time.

Exemplary system and method for obtaining polarization sensitive information is described in U.S. Patent No. 6,208,415. Exemplary OFDI techniques and systems are described in International Application No. PCT/US04/029148. Method and system to determine polarization properties of tissue is described in International Application No. PCT/US05/039374.

Accordingly, there may be a need to address and/or overcome at least some of the deficiencies described herein above.

## SUMMARY OF EXEMPLARY EMBODIMENTS OF THE DISCLOSURE

To overcome at least some of the deficiencies described herein above, the present invention proposes an apparatus and a method the main features of which are defined in the appended claims 1 and 9, respectively. Exemplary embodiments of methodand apparatus according to the present invention can be provided, where two independent polarization states may be simultaneously incident on the sample.

For example, the two incident polarization states are discerned by tagging the two states with different frequency shifts such that the carrier frequencies of the interference fringes are different. Moreover, in the exemplary detection system, apparatus and method, the complex field of the reflected sample arm light is determined independently for each incident polarization state simultaneously. The simultaneous detection of the complex electrical fields and their relative phase can facilitate a determination of, e.g., all 7 independent parameters of the Jones matrix, whereas in prior methods, only, e.g., 5 independent parameters are determined. (See B.H. Park, M.C. Pierce, B. Cense and J.F. de Boer, "Jones matrix analysis for a polarization-sensitive optical coherence tomography system using fiber-optic components," Optics Letters 29(21): 2512-2514 (2004).).

Thus, according to certain exemplary embodiments of the present invention, exemplary systems, apparatus and processes can be provided for determining the non-depolarizing polarization properties (e.g., all 7 independent parameters of the complex 2x2 Jones matrix) of a sample imaged by interferometry with no restrictions on the use of optical fiber or non-diattenuating fiber components, such as circulators and splitters. The exemplary embodiments of the process, software arrangement and system according to the present invention are capable of determining, e.g., all 7 independent parameters of the complex 2x2 Jones matrix between two different locations within the sample probed simultaneously with a minimum of two unique incident polarization states imaged by interferometry. Thus, according to the exemplary embodiments of the present invention, it is possible to:

- determine the full polarization properties of a sample by determining all 7 independent parameters of the complex 2x2 Jones matrix between two different locations within the sample probed simultaneously with a minimum of two unique incident polarization states;
- provide an unrestricted placement of optical fiber and non-diattenuating fiber components throughout a polarization-sensitive interferometric imaging system;
- provide a power efficient interferometer configuration, where the number of optical elements in the sample arm path to the detectors is minimal, providing the most power to the sample, and minimal loss of reflected sample arm light reaching the detectors, and
- determine, e.g., the full sample Jones matrix with no assumptions regarding the optic axes for sample birefringence and diattenuation.

For example, an exemplary embodiment of system, apparatus and procedure according to the present invention can facilitate a determination of the non-depolarizing polarization properties of a sample by comparing the light reflected from two different locations within the sample probed simultaneously with a minimum of two unique incident polarization states in such a way that, e.g., all 7 unique elements of the Jones matrix can be determined.

Further, exemplary embodiments of apparatus, methods and systems according to the present disclosure can be provided for optical frequency domain imaging (e.g., partially fiber-based) to obtain information associated with an anatomical structure or a sample. For example, it is possible to provide at least one first electro-magnetic radiation, e.g., using at least one first arrangement, where a frequency of radiation associated with the first electro-magnetic radiation(s) varies over time. In addition, using at least one second arrangement, it is possible to separate at least one portion of a radiation which is (i) the first electro-magnetic radiation(s) and/or (ii) at least one further radiation into second and third radiations having difference orthogonal states, and to apply at least one first characteristic to the second radiation and at least one second characteristic to at least one third radiation. The first and second characteristics are different from one another.

At least one further electro-magnetic radiation is produced by depolarizing the first electro-magnetic radiation(s) using at least one third arrangement, where the second and third radiations are generated based on the at least one further radiation.

Further, using at least one fourth arrangement, an interference is received and/or detected between (i) at least one fourth radiation and (ii) the second and third radiations, and at least some of Jones matrix elements of a sample are determined based on a radiation reflected from the sample, or possibly, all of the Jones matrix elements of the sample are determined.

The second and third radiations are received and/or detected simultaneously. The radiation reflected from the sample can provided from at least two different locations within the sample which are received simultaneously. The fourth arrangement can be configured to separate the interference into additional radiations having respective first and second polarization states. At least one fifth arrangement can be provided to generate at least one image as a function of at least one of the Jones matrix elements.

The first characteristic(s) is a first frequency shift of the second radiation, and the second characteristic(s) is a second frequency shift of the third radiation. Further, the first and second frequency shifts are different from one another.

The at least one first arrangement is an energy source arrangement. The energy source arrangement is a swept source arrangement which rapidly tunes a wavelength of the first radiation(s).

According to a further exemplary embodiment of the present disclosure, the second arrangement(s) can include at least one acousto-optic modulator arrangement. Further, it is possible to configure the second arrangement(s) to overlap and/or combine the second and third radiations after the first and second characteristics are applied thereto.

The at least one fourth arrangement is further configured to separate the interference into additional radiations having respective first and second polarization states.

These and other objects, features and advantages of the exemplary embodiment of the present disclosure will become apparent upon reading the following detailed description of the exemplary embodiments of the present disclosure, when taken in conjunction with the appended claims.

## BRIEF DESCRIPTION OF THE DRAWING(S)

Further objects, features and advantages of the present invention will become apparent from the following detailed description taken in conjunction with the accompanying figures showing illustrative embodiments of the present disclosure, in which:

Figure 1 is a diagram of an exemplary embodiment of a polarization-sensitive interferometric imaging system/apparatus which can be used with the exemplary software arrangements and processes/methods according to the present disclosure;

Figure 2 is a diagram of an alternative exemplary embodiment of a polarization-sensitive interferometric imaging system/apparatus which can be used with the exemplary software arrangements and processes/methods according to the present disclosure; and

Figures 3(a)-3(g) are exemplary images obtained using the exemplary system/apparatus shown in Figure 1, whereas Figures 3(a) and 3(b) are exemplary images of a chicken muscle, ex-vivo, Figures 3(c) and 3(d) are exemplary images of a human hand top, in-vivo, and Figures 3(e) and 3(f) are exemplary images of a mouse cancer model, in-vivo.

Throughout the figures, the same reference numerals and characters, unless otherwise stated, are used to denote like features, elements, components or portions of the illustrated embodiments. Moreover, while the subject disclosure will now be described in detail with reference to the figures, it is done so in connection with the illustrative embodiments. It is intended that changes and modifications can be made to the described exemplary embodiments without departing from the true scope of the subject disclosure as defined by the appended claims.

## DETAILED DESCRIPTION OF EXEMPLARY EMBODIMENTS

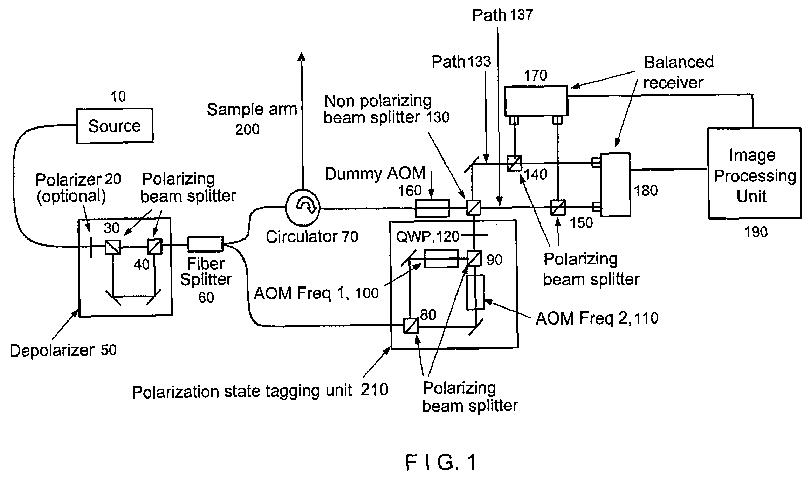

Exemplary embodiments of systems, apparatus, arrangements, software arrangements and processes/methods according to the present disclosure can be implemented in, e.g., a variety of OCT systems. Figure 1 shows an exemplary embodiment of a polarization-sensitive interferometric arrangement that can be used for implementing the exemplary embodiments of the systems, apparatus, arrangements, software arrangements and processes/methods according to the present disclosure.

In particular, as shown in a diagram of Figure 1, the exemplary arrangement of an apparatus and/or system according to the present disclosure can include, e.g., a rapid wavelength tunable source 10 that can be configured to generate an electro-magnetic radiation or light signal. Such radiation and/or light signal can be transmitted through a static polarization controller, and then can enter a depolarizing unit/arrangement 50. Such depolarizing unit/arrangement can include an optional polarizer 20 oriented, e.g., at 45 degrees with respect to a horizontal plane. The light (e.g., or other electro-magnetic radiation) can than be split by a first polarizing beam splitter 30 into, e.g., equal intensities with orthogonal polarization states (e.g., horizontal and vertical). The horizontal and vertical polarization states can each travel along a different path length before a recombination of the beam paths in a second polarizing beam splitter 40. The path length difference between the orthogonal polarization states can preferably be larger than the instantaneous coherence length of the source light/radiation.

After exiting the second polarizing beam splitter 40, the light/radiation can be depolarized with a zero degree of polarization. The light//radiation can be separated into a sample arm component and a reference arm component. The sample arm light/radiation component can be directed to a circulator 70 and a sample arm 200. The reflected light/radiation from the sample can be directed by the circulator to an acousto-optic modulator (AOM) crystal 160 and incident on a non-polarizing beam splitter 130. The reference arm light/radiation can be directed to a polarization tagging state unit/arrangement 210 that can split the unpolarized light/radiation in two portions by, e.g., a polarizing beam splitter 80. The two (or more) portions can receive a frequency shift by AOM Freq 1 100 and AOM Freq 2 110, where the frequency shift introduced by AOM Freq 1 100 can be different from the frequency shift introduced by AOM Freq 2 110.

As shown in the exemplary embodiment of Figure 1, the orthogonal polarizations (e.g., two or more) can be recombined by a polarizing beam splitter 90. The light/radiation can propagate optionally through a Quarter Wave Plate (QWP) 120 and/or via an optical fiber and/or through free space to a non polarizing beam splitter 130 to recombine the sample and reference arm lights/radiations to form interference fringes in beam paths 133, 137. The light/radiation in the beam paths 133, 137 can be split into orthogonal polarization states by, e.g., polarizing beam splitters 140, 150, respectively, and a first balanced receiver 170 can receive the balanced interference signal for one polarization state, and a second balanced receiver 180 can receive the balanced interference for the orthogonal polarization state.

For example, the reference arm light/radiation can be prepared by the QWP 120 and/or a fiber based polarization controller, such that the light intensity that has passed through the AOM Freq 1 100 can be split in, e.g., equal parts in the beam paths 133, 137. Subsequently, the intensity in the four beams after polarizing beam splitters 140 and 150 can all be nearly equal. In addition, the light/radiation intensity that has passed through the AOM Freq 2 110 can be split, e.g., in equal parts in the beam paths 133, 137, and subsequently the intensity in the four beams after polarizing beam splitters 140 and 150 can all be nearly equal. The signals of the balanced receivers can be processed by an image processing unit/arrangement 190 to obtain, e.g., a plurality of (e.g., 7) independent parameters of the complex 2x2 Jones matrix.

A retrieval of sample optical polarization properties and the (e.g., 7) independent parameters of the complex 2x2 Jones matrix can be described in the following manner. After the depolarizer, the light/radiation provided by the source 10 can be unpolarized (e.g., a degree of polarization can be zero).

For example, it is possible to assume the reference arm with AOM Freq 2 110 is blocked by a beam stop. Further, likely only the polarization component of the unpolarized sample arm light/radiation (which is equal to the polarization component transmitted through AOM Freq 1 100) interferes with the reference arm light/radiation. The interference fringes can be centered at the AOM frequency 1 frequency. The balanced detector units/arrangements 170, 180 can detect the orthogonal components of the interference fringes for, e.g., a single sample arm polarization state incident on the sample. A phase sensitive demodulation of the interference fringes centered at AOM frequency 1 can facilitate a determination of the complex electric field components reflected from the sample arm.

Further, with the assumption that the reference arm with AOM Freq 1 100 is blocked by a beam stop, the balanced detector units/arrangements 170, 180 can detect the orthogonal components of the interference fringes for the orthogonal sample arm polarization state incident on the sample, where the interference fringes can be centered at the AOM frequency 2 frequency. In addition, without the beam stops, the sample polarization information can be measured for, e.g., 2 or more sample polarization states simultaneously incident on the sample, where the information for the two polarization states can be centered at a carrier frequency determined by AOM frequency 1 and AOM frequency 2, respectively.

Preferably, the signal bandwidth for each polarization state can be smaller than the frequency difference between AOM frequency 1 and AOM frequency 2. As a result, the complex field components along orthogonal directions for two orthogonal polarization states reflected from the sample arm can be simultaneously measured, e.g., permitting a complete determination of the complex 2x2 Jones matrix.

Referring again to the diagram of the exemplary apparatus/system of Figure 1, the source 10 can be, e.g., a polygonal-scanner based wavelength-swept source. According to one exemplary embodiment, the source 10 can operate at, e.g., 31K axial scans/s with the output of 45 mW, the bandwidth of 1300nm centered at 1295 nm, and its spectral line width of 0.23nm for the depth range of 1.6mm in the air in one side. According to a further exemplary embodiment, the light/radiation from the source 10 can first be forwarded to a depolarizer arrangement (e.g., element/arrangement) 50, where light can be equally split depending on the polarization state and recombined with a sufficient path length delay on one side which can be, e.g., much longer than the coherence length of the source 10.

Further, the recombined light/radiation can be depolarized. After the depolarizer arrangement 50, e.g., 90% of the light/radiation can be forwarded to the sample arm 200 for probing the sample(s), and the rest 10% of the light/radiation can be forwarded to the transmission reference arm. In the transmission reference arm, individual polarization states can be tagged by a polarization state tagging unit/arrangement 210, in which the states can be frequency shifted to, e.g., about 20MHz and 40MHz, respectively, by two or more acousto-optic modulators (AOMs) 100, 110 to utilize both sides of frequency bands, and to, e.g., double the imaging depth range which can become, e.g., about 3.2mm in the air. The light/radiation from the reference transmission arm can be combined with the light/radiation reflected from the sample for interference, and the interference signal can be detected at the balanced receivers 170, 180 in the exemplary polarization-diverse balanced detection configuration. A plurality (e.g., two) channel signals from the exemplary polarization diverse configuration can be acquired simultaneously at an ADC board running at, e.g., about 100MHz sampling frequency, incorporated in the image processing unit/arrangement 190. From the exemplary available signal bandwidth of about 50 MHz, the interference signals of individual incident polarization states can occupy, e.g., two separate detection bands: e.g., one band from about 10MHz to 30MHz, and another one from about 30MHz to 50MHz.

According to a particular exemplary embodiment, the acquired exemplary spectra can contain, e.g., about 3072 pixels in 130nm bandwidth in FWHM. The spectra can be Fourier transformed into the frequency domain, and divided into the two frequency bands. Each frequency band was demodulated, and inverse Fourier transformed to the time domain. Then, the time to k-space mapping can be applied to the spectra based on pre-calibrated wavelength data and interpolation procedure, and the dispersion compensation can be applied based on the pre dispersion measurement due to the difference of dispersion between reference and sample arms. Further, the spectra in equal K-space can be Fourier transformed into reflectivity profiles in depth space. The imaging was performed with a handheld probe with an optical window at the tip. The depth range of the cross-sectional image can be, e.g., about 2.3mm, with consideration of the refractive index of tissues being about 1.4. Exemplary intensity images can be obtained by, e.g., summing intensities of both channels and bands, and polarization sensitive (PS) exemplary images can be obtained as accumulative phase retardation with respect to the surface states, and displayed as black for 0°, and white for 180° phase retardations, and then wrapped back to black for 360°.

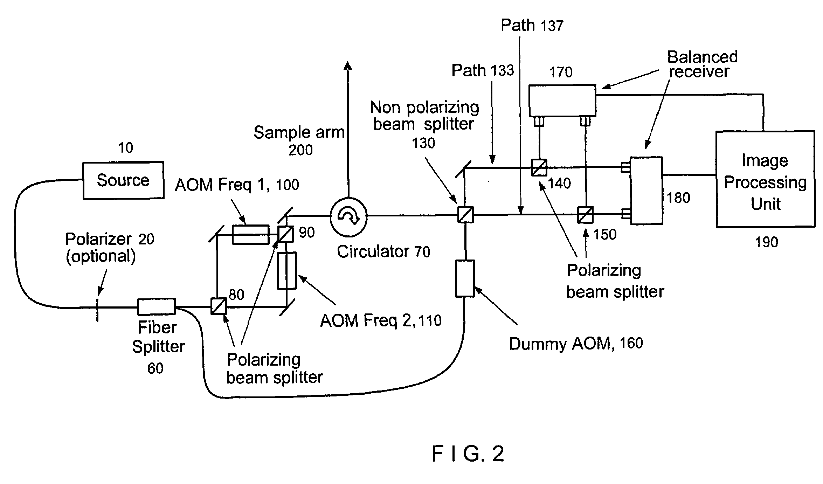

Figure 2 illustrates a diagram of another exemplary embodiment of the system/apparatus according to the present disclosure which can accomplish same or similar goals and/or results as the exemplary embodiment illustrated in Figure 1. With respect to Figure 2, the depolarizing element/arrangement 50 can be excluded, and the tagging of orthogonal independent polarization states can be accomplished in the sample arm using element/arrangements 80, 90, 100, 110, where these elements/arrangement can be similar, equal to or same as those described herein above.

The exemplary embodiment of a PS analysis method according to exemplary embodiment of the present disclosure can be based on Jones matrix. The non-depolarizing polarization properties of an exemplary optical system/apparatus can be described by its complex Jones matrix, J, which transforms an incident polarization state, described by a complex electric field vector, $\mathbf{E}=[HV]^T$, to a transmitted state, $\mathbf{E'}=[H'V]^T$. In the PS-OCT analysis method based on the Jones matrix, the measurement of polarization states within the sample, $\left[H_1' V_1'\right]^T$ , $\left[H_2' V_2'\right]^T$ with respect to the surface polarization states, $[H_1 V_1]^T$, $[H_2 V_2]^T$ is formulated as,

$$\left[H_1'\ H_2';V_1'\ V_2'\right] = \exp(i\Delta\psi_1)\times\mathbf{J}_{\text{out}}\mathbf{J}_{\text{S}}\mathbf{J}_{\text{out}}^{-1}\left[H_1\ \exp(i\alpha)H_2;V_1\ \exp(i\alpha)V_2\right],$$

where $\mathbf{J}_{\text{out}}$ describes the optical path from the sample surface to the detectors, and is modeled as elliptical retarders. $\mathbf{J}_{\text{S}}$ describes the round-trip Jones matrix of the sample, and can be decomposed into a form of $\mathbf{J}_{\text{S}} = \mathbf{J}_{\text{R}}\mathbf{J}_{\text{P}}$ where $\mathbf{J}_{\text{R}}$ and $\mathbf{J}_{\text{P}}$ describe a retarder and a polarizer respectively. $\alpha$ is the phase difference between the measurements with two incident polarization states. Since the two measurements can be simultaneous in the exemplary configuration, there is likely no ambiguity in phase and $\alpha$ become zero, $\alpha = 0$. The above-described formula become simplified as follows:

$$\mathbf{J}_{\text{T}} = \exp(-i\Delta\psi_1)\times\left[H_1'\ H_2';V_1'\ V_2'\right]\times\left[H_1\ H_2;V_1\ V_2\right]^{-1},$$

where $\mathbf{J}_{\text{T}}$ is a combined Jones matrix including the output path, $\mathbf{J}_{\text{T}} = \mathbf{J}_{\text{out}}\mathbf{J}_{\text{S}}\mathbf{J}_{\text{out}}^{-1}$. This gives the full Jones matrix which contains all the information of the polarization properties of the sample.

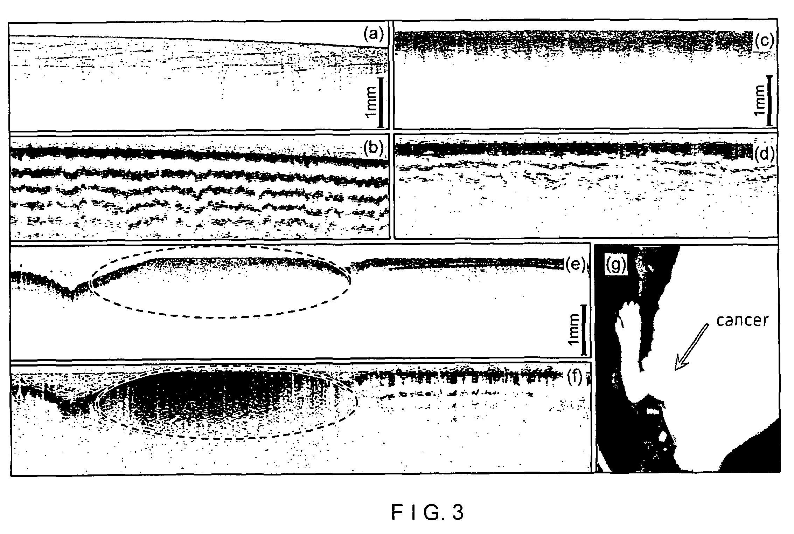

In order to demonstrate the implementation of the exemplary embodiments of the method, apparatus and system according to the present disclosure, samples of chicken thigh muscles were imaged as ex-vivo, and the back sides of a human hand were imaged in vivo as shown in Figures 3(a)-3(f). Dimensions of cross-sectional images were 2.3mm x 8mm in the tissue depth and lateral directions respectively. The exemplary intensity image of the chicken muscle as provided in Figure 3(a) shows its structures with slow intensity decay with the depth compared with other biological tissues, and the exemplary PS image of Figure 3(b) shows frequent horizontal black-white banding patterns down to bottom of the image. The exemplary intensity image of the hand in Figure 3(c) shows the superficial epithelium, and the underlying dermis structures, and the exemplary PS image of Figure 3(d) shows some birefringence. As shown in Figures 3(c) and 3(d), the back side of the hand showed stronger birefringence than the other side. The PS imaging is known to provide additional contrast to distinguish between normal and cancerous tissues in case the normal tissue is birefringent.

To demonstrate such exemplary procedure and implementation in the animal model, a mouse cancer model was imaged with the exemplary embodiment of a PS-OFDI system in accordance with the present disclosure. Cancer cells were injected into the back legs of mice superficially, and the exemplary PS-OFDI imaging was performed from day 1 longitudinally until day 10. Since the cancer was injected just under the skin at the location of muscle, PS-OFDI imaging showed some distinction of the cancer region from the normal muscle tissue. Dimensions of cross-sectional images were 2.3mm x 12mm in the tissue depth, and lateral directions respectively. Both the exemplary intensity and PS images of Figures 3(e) and 3(f) shows a distinction of the cancer tissue from the surrounding tissue: the cancer tissue appears as relatively homogeneous structures without banding pattern indicating no birefringence. It appears that the cancer section has clear boundaries separating from normal tissue sections without metastasis.

The foregoing merely illustrates the principles of the invention. Various modifications and alterations to the described embodiments will be apparent to those skilled in the art in view of the teachings herein. Indeed, the arrangements, systems and methods according to the exemplary embodiments of the present invention can be used with imaging systems, and for example with those described in International Patent Publication WO 2005/047813 published May 26, 2005, U.S. Patent Publication No. 2006/0093276, published May 4, 2006, and U.S. Patent Publication No. 2005/0018201, published January 27, 2005.

It will thus be appreciated that those skilled in the art will be able to devise numerous systems, arrangements and methods which, although not explicitly shown or described herein, embody the principles of the invention and are thus within the scope of the present invention as defined by the claims.

**Claims**

1. A polarization-sensitive optical coherence tomography apparatus for determining non-depolarizing polarization parameters of a sample, comprising:

   at least one frequency swept light source first arrangement (10) configured to provide at least one first electro-magnetic light radiation, wherein a frequency of radiation provided by the at least one first arrangement (10) varies over time;
   a depolarizer (50) arranged for depolarizing the at least one first electro-magnetic light radiation provided from the first arrangement to generate at least one depolarized electromagnetic light radiation;
   at least one splitter second arrangement (60, 80,130) comprising (i) a first splitter (60) configured to split the at least one depolarized electromagnetic light radiation into second
   depolarized light radiation provided to a sample and third depolarized light radiation provided to a reference, and (ii) a polarizing second splitter (80) arranged to split the third depolarizing light radiation into at least two orthogonally polarized reference light components;
   modulation means (100, 110) configured to apply different frequency modulations to the reference light components, wherein the at least one splitter second arrangement (60, 80, 130) is further configured to combine a fourth electromagnetic light, resulting from the second light radiation returning from the sample , and the modulated reference light components into an interference light radiation;
   detection means (170, 180) configured to simultaneously detect different polarization orthogonal components of the interference light radiation; and
   signal processing means (190) configured to determine Jones matrix elements associated with the sample based on the detected polarization orthogonal components of the interference light radiation.

2. The apparatus according to claim 1, wherein the signal processing means (190) is configured to determine all of the Jones matrix elements of the sample.

3. The apparatus according to claim 1, wherein the fourth electromagnetic light radiation returning from the sample is reflected from the sample and is provided from at least two different locations within the sample which are detected simultaneously.

4. The apparatus according to claim 1, wherein the modulation means (100, 110) applies the different frequency modulations by respective first and second frequency shifts.

5. The apparatus according to claim 4 , wherein the first and second frequency shifts are different from one another.

6. The apparatus according to claim 1, wherein the modulation means (100, 110) includes at least one acousto-optic modulator.

7. The apparatus according to claim 1, wherein the detection means (170, 180) is further configured to separate the interference light radiation into additional radiations having respective first and second polarization states.

8. The apparatus according to claim 1, further comprising at least one image processing fifth arrangement (190) configured to generate at least one image as a function of at least one of the Jones matrix elements.

9. A polarization-sensitive optical coherence tomography method for determining non-depolarizing polarization parameters of a sample, comprising:

   providing at least one first electro-magnetic light radiation, radiation from at least one frequency swept light source first arrangement (10), wherein a frequency of radiation associated with the at least one first radiation varies over time;
   depolarizing the at least one first electro-magnetic light radiation provided from the first arrangement (10) to generate at least one depolarized electromagnetic light radiation;
   splitting the at least one depolarized electromagnetic light radiation with at least one splitter second arrangement (60, 80, 130) into second depolarized light radiation provided to a sample and third depolarized light radiation provided to a reference, splitting the third depolarizing light radiation into at least two orthogonally polarized reference light components;
   applying different frequency modulations to the reference light components, wherein the at least one splitter second arrangement is further configured combine a fourth electromagnetic light radiation, resulting from the second light radiation returning from the sample, and the modulated reference light components into an interference light radiation;

simultaneously detecting different polarization orthogonal components of the interference light radiation; and determining Jones matrix elements associated with the sample based on the detected polarization orthogonal components of the interference light radiation.

10. The method according to claim 9 , wherein the determining procedure comprising determining all of the Jones matrix elements of the sample.

**Patentansprüche**

1. Eine polarisationsempfindliche optische Kohärenztomografievorrichtung zum Bestimmen nicht-depolarisierender Polarisationsparameter einer Probe, die folgende Merkmale aufweist:

zumindest eine erste Anordnung (10) einer frequenzgewobbelten Lichtquelle, die ausgebildet ist, um zumindest eine erste elektromagnetische Lichtstrahlung bereitzustellen, wobei eine Frequenz der Strahlung, die durch die zumindest eine erste Anordnung (10) bereitgestellt wird, im Lauf der Zeit variiert;

einen Depolarisator (50), der angeordnet ist zum Depolarisieren der zumindest einen ersten elektromagnetischen Lichtstrahlung, die von der ersten Anordnung bereitgestellt wird, um zumindest eine depolarisierte elektromagnetische Lichtstrahlung zu erzeugen;

zumindest eine zweite Anordnung von Aufteilern (60, 80, 130), die (i) einen ersten Aufteiler (60), der ausgebildet ist, um die zumindest eine depolarisierte elektromagnetische Lichtstrahlung in eine zweite depolarisierte Lichtstrahlung, die einer Probe bereitgestellt wird, und eine dritte depolarisierte Lichtstrahlung, die an einer Referenz bereitgestellt wird, aufzuteilen, und (ii) einen polarisierenden zweiten Aufteiler (80) aufweist, der angeordnet ist, um die dritte depolarisierende Lichtstrahlung in zumindest zwei orthogonal polarisierte Referenzlichtkomponenten aufzuteilen;

eine Modulationseinrichtung (100, 110), die ausgebildet ist, um unterschiedliche Frequenzmodulationen an die Referenzlichtkomponenten anzulegen, wobei die zumindest eine zweite Anordnung von Aufteilern (60, 80, 130) ferner ausgebildet ist, um ein viertes elektromagnetisches Licht, das sich von der zweiten Lichtstrahlung ergibt, die von der Probe zurückkehrt, und die modulierten Referenzlichtkomponenten in eine Interferenzlichtstrahlung zu kombinieren;

eine Erfassungseinrichtung (170, 180), die ausgebildet ist, um gleichzeitig unterschiedliche Polarisationsorthogonalkomponenten der Interferenzlichtstrahlung zu erfassen; und

eine Signalverarbeitungseinrichtung (190), die ausgebildet ist, um basierend auf den erfassten Polarisationsorthogonalkomponenten der Interferenzlichtstrahlung Jones-Matrix-Elemente zu bestimmen, die der Probe zugeordnet sind.

2. Die Vorrichtung gemäß Anspruch 1, bei der die Signalverarbeitungseinrichtung (190) ausgebildet ist, um alle Jones-Matrix-Elemente der Probe zu bestimmen.

3. Die Vorrichtung gemäß Anspruch 1, bei der die vierte elektromagnetische Lichtstrahlung, die von der Probe zurückkehrt, von der Probe reflektiert wird und von zumindest zwei unterschiedlichen Stellen in der Probe bereitgestellt wird, die gleichzeitig erfasst werden.

4. Die Vorrichtung gemäß Anspruch 1, bei der die Modulationseinrichtung (100, 110) die unterschiedlichen Frequenzmodulationen durch jeweilige erste und zweite Frequenzverschiebungen anlegt.

5. Die Vorrichtung gemäß Anspruch 4, bei der die erste und zweite Frequenzverschiebung unterschiedlich zueinander sind.

6. Die Vorrichtung gemäß Anspruch 1, bei der die Modulationseinrichtung (100, 110) zumindest einen akustooptischen Modulator umfasst.

7. Die Vorrichtung gemäß Anspruch 1, bei der die Erfassungseinrichtung (170, 180) ferner ausgebildet ist, um die Interferenzlichtstrahlung in zusätzliche Strahlungen zu trennen, die einen jeweiligen ersten und zweiten Polarisationszustand aufweisen.

**8.** Die Vorrichtung gemäß Anspruch 1, die ferner zumindest eine fünfte Bildverarbeitungsanordnung (190) aufweist, die ausgebildet ist, um zumindest ein Bild als eine Funktion von zumindest einem der Jones-Matrix-Elemente zu erzeugen.

**9.** Ein polarisationsempfindliches optisches Kohärenztomografieverfahren zum Bestimmen nicht-depolarisierender Polarisationsparameter einer Probe, das folgende Schritte aufweist:

Bereitstellen zumindest einer ersten elektromagnetischen Lichtstrahlung, Strahlung von zumindest einer ersten Anordnung (10) einer frequenzgewobbelten Lichtquelle, wobei eine Frequenz der Strahlung, die der zumindest einen ersten Strahlung zugeordnet ist, im Lauf der Zeit variiert;

Depolarisieren der zumindest einen ersten elektromagnetischen Lichtstrahlung, die von der ersten Anordnung (10) bereitgestellt wird, um zumindest eine depolarisierte elektromagnetische Lichtstrahlung zu erzeugen;

Aufteilen der zumindest einen depolarisierten elektromagnetischen Lichtstrahlung mit zumindest einer zweiten Anordnung von Aufteilern (60, 80, 130) in eine zweite depolarisierte Lichtstrahlung, die einer Probe bereitgestellt wird, und eine dritte depolarisierte Lichtstrahlung, die einer Referenz bereitgestellt wird,

Aufteilen der dritten depolarisierenden Lichtstrahlung in zumindest zwei orthogonal polarisierte Referenzlichtkomponenten;

Anlegen unterschiedlicher Frequenzmodulationen an die Referenzlichtkomponenten, wobei die zumindest eine zweite Anordnung von Aufteilern ferner ausgebildet ist, um eine vierte elektromagnetische Lichtstrahlung, die sich von der zweiten Lichtstrahlung ergibt, die von der Probe zurückkehrt, und die modulierten Referenzlichtkomponenten in eine Interferenzlichtstrahlung zu kombinieren;

gleichzeitiges Erfassen unterschiedlicher Polarisationsorthogonalkomponenten der Interferenzlichtstrahlung; und

Bestimmen von Jones-Matrix-Elementen, die der Probe zugeordnet sind, basierend auf den erfassten Polarisationsorthogonalkomponenten der Interferenzlichtstrahlung.

**10.** Das Verfahren gemäß Anspruch 9, bei dem der Bestimmungsvorgang das Bestimmen aller Jones-Matrix-Elemente der Probe aufweist.

**Revendications**

**1.** Appareil de tomographie par cohérence optique sensible à la polarisation comprenant :

au moins une première disposition de sources lumineuses à balayage de fréquence (10) configurée pour fournir au moins un premier rayonnement électromagnétique lumineux, une fréquence de rayonnement fournie par l'au moins une première disposition (10) variant dans le temps ;

un dépolarisant (50) disposé pour dépolariser l'au moins un premier rayonnement électromagnétique lumineux fourni par la première disposition pour générer au moins un rayonnement électromagnétique lumineux dépolarisé ;

au moins une seconde disposition de séparateurs (60, 80, 130) comprenant (i) un premier séparateur (60) configuré pour scinder l'au moins un rayonnement électromagnétique lumineux dépolarisé en un deuxième rayonnement lumineux dépolarisé et un troisième, et (ii) un second séparateur polarisant (80) disposé pour scinder le troisième rayonnement lumineux dépolarisé en au moins deux composantes lumineuses de référence polarisées orthogonalement ;

des moyens de modulation (100, 110) configurés pour appliquer différentes modulations de fréquence aux composantes lumineuses de référence, l'au moins une seconde disposition de séparateurs (60, 80, 130) étant en outre configurée pour combiner un quatrième rayonnement électromagnétique lumineux issu du renvoi du second rayonnement lumineux par l'échantillon et les composantes lumineuses de référence modulées pour donner un rayonnement lumineux d'interférence ;

des moyens de détection (170, 180) configurés pour détecter différentes composantes orthogonales de polarisation du rayonnement lumineux d'interférence ; et

des moyens de traitement du signal (190) configurés pour déterminer les éléments d'une matrice de Jones associés à l'échantillon en se basant sur les composantes orthogonales de polarisation détectées du rayonnement lumineux d'interférence.

**2.** Appareil selon la revendication 1, dans lequel le moyen de traitement du signal (190) est configuré pour déterminer tous les éléments de matrice de Jones de l'échantillon.

**3.** Appareil selon la revendication 1, dans lequel le quatrième rayonnement électromagnétique lumineux renvoyé par l'échantillon est réfléchi par l'échantillon et provient d'au moins deux endroits différents de l'échantillon qui sont détectés simultanément.

**4.** Appareil selon la revendication 1, dans lequel les moyens de modulation (100, 110) appliquent les différentes modulations de fréquence par un premier et un second décalages de fréquence respectivement.

**5.** Appareil selon la revendication 4, dans lequel les premier et second décalages de fréquence sont différents l'un de l'autre.

**6.** Appareil selon la revendication 1, dans lequel les moyens de modulation (100, 110) comprennent au moins un modulateur optoacoustique.

**7.** Appareil selon la revendication 1, dans lequel les moyens de détection (170, 180) sont en outre configurés pour séparer le rayonnement lumineux d'interférence en rayonnements additionnels ayant des premier et second états de polarisation respectifs.

**8.** Appareil selon la revendication 1, comprenant en outre au moins une cinquième disposition de traitement d'image (190) configurée pour générer au moins une image en fonction d'au moins un des éléments de la matrice de Jones.

**9.** Procédé de tomographie par cohérence optique sensible à la polarisation destiné à déterminer les paramètres de polarisation non dépolarisants d'un échantillon, consistant à :

fournir au moins un premier rayonnement électromagnétique lumineux provenant d'au moins une première disposition de sources lumineuses (10) à balayage de fréquence, une fréquence de rayonnement associée à l'au moins un premier rayonnement variant dans le temps ;

dépolariser l'au moins un premier rayonnement électromagnétique lumineux fourni par la première disposition (10) pour générer au moins un rayonnement électromagnétique lumineux dépolarisé ;

scinder l'au moins un rayonnement électromagnétique lumineux dépolarisé avec au moins une seconde disposition de séparateurs (60, 80, 130) en un deuxième rayonnement lumineux émis vers un échantillon et un troisième rayonnement lumineux dépolarisé émis vers une référence ;

scinder le troisième rayonnement lumineux dépolarisé en au moins deux composantes lumineuses de référence polarisées orthogonalement ;

appliquer différentes modulations de fréquence aux composantes lumineuses de référence, l'au moins une seconde disposition de séparateurs étant en outre configurée pour combiner un quatrième rayonnement électromagnétique lumineux résultant du renvoi par l'échantillon du second rayonnement lumineux et les composantes lumineuses de référence modulées en un rayonnement lumineux d'interférence ;

détecter simultanément les différentes composantes orthogonales de polarisation du rayonnement d'interférence lumineux ;

et

déterminer les éléments de matrice de Jones associés à l'échantillon, en se basant sur les composantes orthogonales détectées du rayonnement lumineux d'interférence.

**10.** Procédé selon la revendication 9, dans lequel la procédure de détermination consiste à déterminer tous les éléments de matrice de Jones de l'échantillon.

F I G. 1

F I G. 2

EP 2 341 823 B1

FIG.3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2007024860 A1
- WO 03062802 A2
- US 514769 P
- US 6208415 B
- US 04029148 W
- US 05039374 W
- WO 2005047813 A
- US 20060093276 A
- US 20050018201 A

### Non-patent literature cited in the description

- **W.Y.OH et al.** High-speed polarization sensitive optical frequency domain imaging with frequency multiplexing. *Optics Express,* 21 January 2008, vol. 16 (2
- **HUANG et al.** Optical Coherence Tomography. *Science,* 1991, vol. 254, 1178
- **SAXER et al.** High-speed fiber-based polarization-sensitive optical coherence tomography of in vivo human skin. *Opt. Lett.,* 2000, vol. 25, 1355
- **J.F. DE BOER et al.** Determination of the depth-resolved Stokes parameters of light backscattered from turbid media by use of polarization-sensitive optical coherence tomography. *Opt. Lett.,* 1999, vol. 24, 300
- **B.H. PARK et al.** In vivo burn depth determination by high-speed fiber-based polarization sensitive optical coherence tomography. *J. Biomed. Opt.,* 2001, vol. 6, 474
- **S.L. JIAO et al.** Two-dimensional depth-resolved Mueller matrix of biological tissue measured with double-beam polarization-sensitive optical coherence tomography. *Opt. Lett.,* 2002, vol. 27, 101
- **S. JIAO et al.** Optical-fiber-based Mueller optical coherence tomography. *Opt. Lett.,* 2003, vol. 28, 1206
- **S.L. JIAO et al.** Depth-resolved two-dimensional Stokes vectors of backscattered light and Mueller matrices of biological tissue measured with optical coherence tomography. *Appl. Opt.,* 2000, vol. 39, 6318
- **S.L. JIAO ; L.V. WANG.** Jones-matrix imaging of biological tissues with quadruple-channel optical coherence tomography. *J. Biomed. Opt.,* 2002, vol. 7, 350
- **B.H. PARK ; M.C. PIERCE ; B. CENSE ; J.F. DE BOER.** Jones matrix analysis for a polarization-sensitive optical coherence tomography system using fiber-optic components. *Optics Letters,* 2004, vol. 29 (21), 2512-2514